(19) Europäisches Patentamt
European Patent Office
Office européen des brevets

(11) Veröffentlichungsnummer: **0 088 938**
**B1**

# (12) EUROPÄISCHE PATENTSCHRIFT

(45) Veröffentlichungstag der Patentschrift: **30.04.86**

(21) Anmeldenummer: **83101999.7**

(22) Anmeldetag: **02.03.83**

(51) Int. Cl.⁴: **C 07 D 213/80**, C 07 D 213/85, C 07 D 491/04, A 61 K 31/455 // (C07D491/04, 307:00, 221:00)

## (54) Verwendung von Pyridinverbindungen zur Herstellung von Arzneimitteln mit antihypoxischer und ischämie-protektiver Wirkung.

(30) Priorität: **13.03.82 DE 3209276**

(43) Veröffentlichungstag der Anmeldung: **21.09.83 Patentblatt 83/38**

(45) Bekanntmachung des Hinweises auf die Patenterteilung: **30.04.86 Patentblatt 86/18**

(84) Benannte Vertragsstaaten: **AT BE CH DE FR GB IT LI LU NL SE**

(56) Entgegenhaltungen:
EP - A - 0 042 569
DE - A - 2 629 892
FR - A - 1 440 647
GB - A - 1 396 681
US - A - 3 907 808

ARZNEIMITTELFORSCHUNG, Band 32, Nr. 4, April 1982, Seiten 338-346, Editio Cantor KG, Aulendorf, DE. R.JOURNAL OF MEDICINAL CHEMISTRY, Band 17, Nr. 9, 1974, Seiten 956-965, Columbus, Ohio, USA B. LOEV et a TOWART et al.: "The effects of nimodipine, its optical isomers and metabolites on isolated vascular sl.: "Hantzsch-Type" dihydropyridine hypotensive agents" mooth muscle"
ARZNEIMITTELFORSCHUNG, Band 22, Nr. 1, 1972, Seiten 60-62, Editio Cantor KG, Aulendorf, DE. K. SCHLOSSMANN: "Fluorometrische Bestimmung des 4-2'-Nitrophenyl)-2,6-dimethyl-1,4-dihydropyridin-3,5-dicarbonsäuredimethylester und seines Hauptmetaboliten"
JOURNAL OF THE CHEMICAL SOCIETY, PERKIN TRANSACTIONS I, 1975, Seiten 926-929, Columbus, Ohio, USA T. CHENNAT et al.: "A new synthesis of 1,4-dihydropyridines"
CHEMICAL ABSTRACTS, Band 89, Nr. 5, 31. Juli 1978, Seite 596, Nr. 43046z, Columbus, Ohio, USA U.M. DZHEMILEV et al.: "New method for the dehydrogenation of 1,4-dihydropyridines to pyridines in the presence of homogeneous complex palladium catalysts".
CHEMICAL ABSTRACTS, Band 92, Nr. 21, 26. Mai 1980, Seite 11, Nr. 174141m, Columbus, Ohio, USA S. KONDO et al.: "Identification of nifedipine metabolites and their determination by gas chromatography"

(73) Patentinhaber: **BAYER AG, Konzernverwaltung RP Patentabteilung, D-5090 Leverkusen 1 Bayerwerk (DE)**

(72) Erfinder: **Wehinger, Egbert, Dr., Gellertweg 33, D-5600 Wuppertal 1 (DE)**
Erfinder: **Meyer, Horst, Dr., Henselweg 11, D-5600 Wuppertal 1 (DE)**
Erfinder: **Benz, Ulrich, Gustav-Poensgenstrasse 29, D-4000 Düsseldorf 1 (DE)**

(56) Entgegenhaltungen: (Fortsetzung)
CHEMICAL ABSTRACTS, Band 84, Nr. 25, 21. Juni 1976, Seite 17, Nr. 173595k, Columbus, Ohio, USA K. SCHLOSSMANN et al.: "Investigations on the metabolism and protein binding of nifedipine"
CHEMICAL ABSTRACTS, Band 79, Nr. 23, 10. Dezember 1973, Seite 6, Nr. 13381f, Columbus, Ohio, USA S.S. WALKENSTEIN et al.: "Metabolism of the antihypertensive agent 1,4-dihydro-2,6-dimethyl-4-(2-trifluoromethylphenyl)-3,5-pyridinedicarboxylic acid diethyl ester"
CHEMICAL ABSTRACTS, Band 68, Nr. 25, 17. Juni 1968, Seite 11019, Nr. 114376m, Columbus, Ohio, USA N.S. PROSTAKOV et al.: "Substituted pyridines. Preparation of 3,5-dimethyl-4-phenylpyridine and synthesis on its basis"
CHEMICAL ABSTRACTS, Band 68, Nr. 21, 20. Mai 1968, Seite 9222, Nr. 95651d, Columbus, Ohio, USA TETSUJI KAMETANI et al.: "Syntheses of heterocyclic compounds, CXCVIII. Streptonigrin and related compounds. 7. Synthesis of the streptonigrin nucleus by the formation of a pyridine ring"
JOURNAL OF HETEROCYCLIC CHEMISTRY, Band 11, Oktober 1974, Seiten 819-821, Columbus, Ohio, USA P.M. CARBATEAS et al.: "Two methods for conversion of an aromatic aldehyde to a 4-arylpyridine. A method for preparation of 3-alkyl-4-arylpyridines"
JOURNAL OF THE CHEMICAL SOCIETY, Nr. 191, 1946, Seiten 884-888, Columbus, Ohio, USA V.A. PETROW: "New syntheses of heterocyclic compounds. Part VII. 9-amino-6: 8-dimethyl-7:10-diazaphenanthreses"
JOURNAL OF MEDICINAL CHEMISTRY, Band 16, Nr. 1, 1973, Seiten 34-37, Columbus, Ohio, USA S.E. PARKER et al.: "Bistransformation of 1,4-Dihydro-2,6 dimethyl-4-(2-trifluoromethylphenyl)-3,5-pyridinedicarboxylic acid diethyl ester"

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

EP 0 088 938 B1

**Beschreibung**

Die vorliegende Erfindung betrifft die Verwendung von teilweise bekannten substituierten Pyridinderivaten zur Herstellung von Arzneimitteln zur Bekämpfung von Schädigungen des Zentralnervensystems, sowie neue Verbindungen aus dieser Stoffgruppe und deren Herstellung.

Es ist bereits bekannt geworden, daß man 2,6-Dimethyl-4-(2-nitrophenyl)-pyridin-3,5-dicarbonsäurediethylester durch Chromsäureoxidation des entsprechenden 1,4-Di-hydro-2,6-dimethyl-4-(2-nitrophenyl)-pyridin-3,5-dicarbonsäurediethylesters erhält (s. V.A. Petrow, J.Chem.Soc. 884 (1946)).

Weiterhin ist bekannt geworden, daß bei der Biotransformation von vasodilatierend wirksamen 4-Aryl-1,4-dihydropyridinderivaten Pyridine entstehen, die wesentlich schwächer gefäßwirksam sind als die entsprechenden Dihydropyridinverbindungen (s. S. Higuchi et al. 95. General Congress of the Japanese Pharmaceutical Society, April 1975; S.E. Parker und J. Weinstock, J.Med.Chem. 16, 34 (1973)).

Weiterhin ist bekannt geworden, daß die Verbindung 1,4-Dihydro-2,6-dimethyl-4-(3-nitrophenyl)-pyridin-3,5-dicarbonsäure-isopropyl-(2-methoxyethyl)-ester bei der Bekämpfung von cerebralen Insuffizienzen eingesetzt werden kann (vgl. DOS 2 815 578).

Weiterhin sind 4-(3-Nitrophenyl)-pyridin-3,5-dicarbon-säureester als Zwischenprodukte bei der Synthese von antibakteriell wirksamen Pyridylchinolonen verwendet worden (P.M. Carbateas und G.L. Williams, J. Heterocyclic Chem. 11, 819 (1974)).

Die Verwendung von Pyridinderivaten der allgemeinen Formel (I) als pharmazeutische Wirkstoffe zur Bekämpfung von Schädigungen des Zentralnervensystems, die auf einer Ischämie und/oder Hypoxie beruhen, ist jedoch neu und bisher noch nicht bekannt geworden.

Die vorliegende Erfindung betrifft die Verwendung von Verbindungen der allgemeinen Formel

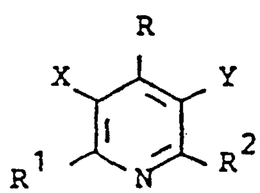

(I)

in welcher

R für Phenyl oder Pyridyl steht, wobei der Phenylrest gegebenenfalls 1- oder 2-fach substituiert ist durch Nitro, Cyano, Trifluormethyl, Fluor, Chlor, Brom, Jod, Alkyl, Alkoxy, Alkylmercapto mit jeweils 1 bis 2 C-Atomen in den Alkyl- und Alkoxyresten,

$R^1$ und $R^2$ gleich oder verschieden sind und jeweils für Wasserstoff cder einen Alkylrest mit 1 bis 4 Kohlenstoffatomen stehen, der gegebenenfalls durch Hydroxy substituiert ist,

oder

$R^1$ zusammen mit X oder $R^2$ zusammen mit Y gemeinsam einen 5- bis 7-gliedrigen Ring bilden der durch Carbonyl und gegebenenfalls durch Sauerstoff, und/ oder NH unterbrochen ist und

X und Y gleich oder verschieden sind und jeweils für eine Nitrilgruppe stehen

oder

für den Rest $-COR^3$ stehen, wobei $R^3$ einen Alkylrest mit 1 bis 4 Kohlenstoffatomen, Phenyl oder Benzyl bedeutet

oder

für die Gruppe $-COOR^4$ stehen, wobei $R^4$ Wasserstoff, für einen geradkettigen, verzweigten oder cyclischen gesättigten oder ungesättigten Kohlenwasserstoffrest mit bis zu 10 Kohlenstoffatomen bedeutet, der gegebenenfalls durch 1 Sauerstoffatom in der Kette unterbrochen ist und/oder der gegebenenfalls substituiert ist durch Hydroxy, Fluor, Chlor, Brom, Cyano, Acetoxy, Pyridyl, Phenyl oder Phenoxy, wobei die Phenylgruppen ihrerseits gegebenenfalls durch Fluor, Chlor, Trifluormethyl, Nitro, Alkyl mit 1 bis 4 C-Atomen oder Alkoxy mit 1 bis 4 C-Atomen substituiert sein können

oder

für die Gruppe $-SO^2R^5$ stehen, wobei $R^5$ einen Alkylrest mit 1 bis 4 Kohlenstoffatomen oder eine Phenylgruppe darstellt, die gegebenenfalls durch Fluor, Chlor, Trifluormethyl, Alkyl oder Alkoxy mit jeweils 1 bis 2 Kohlenstoffatomen substituiert ist,

zur Herstellung eines Arzneimittels zur Bekämpfung von Schädigungen des zentralen Nervensystems, sowie eine neue Verbindung und deren Herstellung.

Überraschenderweise zeigen die erfindungsgemäßen Verbindungen eine starke Hypoxie-Schutzwirkung, die sowohl bei einer Hypoxie-induzierten Amnesie als auch bei der Hypoxietoleranz zum Ausdruck kommt. Von den aus dem Stand der Technik bekannten, substituierten Pyridinverbindungen sind diese speziellen pharmakologischen Wirkungen bisher nicht bekannt geworden, so daß die erfindungsgemäßen Verbindungen hinsichtlich dieser Eigenschaften als Bereicherung der Pharmazie anzusehen sind.

Die Verbindungen sind entweder bekannt oder können in einfacher Weise hergestellt werden, indem man

a) 1,4-Dihydropyridinderivate der allgemeinen Formel (II)

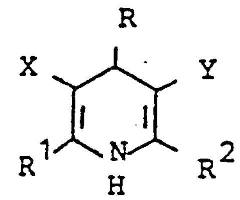

(II)

in welcher

R, $R^1$, $R^2$, X und Y die oben angegebene Bedeutung haben, mit oxidierenden (dehydrierenden) Agentien, gegebenenfalls in Gegenwart inerter Lösungsmittel, bei Temperaturen zwischen 0 und 200°C zur Reaktion bringt, wobei als Oxidationsmittel (Dehydrierungsmittel) in erster Linie Salpetersäure oder salpetrige Säure, Chrom-(VI)-oxid oder Natriumdichromat, Stickoxide, Chloranil, Tetracyanobenzochinon oder die anodische Oxidation in Gegenwart eines geeigneten Elektrolytsystems in Frage kommen,

oder indem man

b) die erfindungsgemäßen Verbindungen gegebenenfalls wechselseitig ineinander überführt, indem-man erfindungsgemäße Verbindungen der Formel (III) mit $R^4 \neq H$

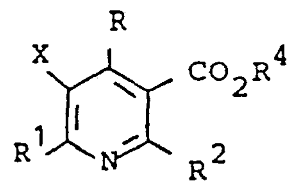

(III)

unter den Bedingungen der alkalischen Hydrolyse in erfindungsgemäße Verbindungen der Formel (III) mit $R^4 = H$ überführt oder umgekehrt erfindungsgemäße Verbindungen der Formel (III) mit $R^4 = H$ nach den literaturbekannten Methoden der Veresterung von Carbonsäuren zu Verbindungen der Formel (III) mit $R^4 \neq H$ umsetzt,

oder indem man

c) erfindungsgemäße Substanzen der allgemeinen Formel (IV),

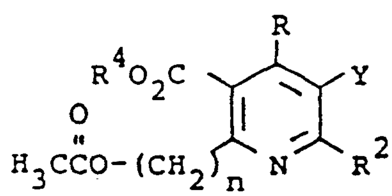

(IV)

in welcher

R, $R^2$, $R^4$ und Y die oben angegebene Bedeutung haben und

n für eine ganze Zahl von 1 bis 4 steht,

nach bekannten Methoden zu Verbindungen der Formel (V)

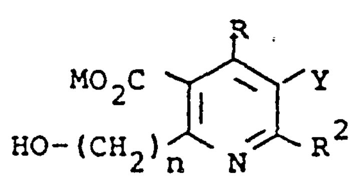

(V)

in welcher

R, $R^2$, Y und die oben angegebene Bedeutung haben

und

M für Alkali steht,

verseift, wobei in saurem Medium das entsprechende Lacton der allgemeinen Formel (VI)

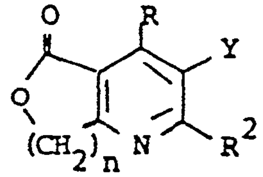

(VI)

entsteht.

Die vorstehenden Herstellungsverfahren sind lediglich zur Verdeutlichung angegeben, und die Herstellung der Verbindungen der Formel (I) ist nicht auf diese Verfahren beschränkt.

Außer den unten angeführten Beispielen seien noch folgende erfindungsgemäßen Wirkstoffe genannt:

2,6-Dimethyl-4-(2-nitrophenyl)-pyridin-3,5-dicarbonsäure-(2-hydroxyethyl)-methyl-ester,
2,6-Dimethyl-4-(2-nitrophenyl)-pyridin-3,5-dicarbonsäure-(2-hydroxyethyl)-ethyl-ester,
2,6-Dimethyl-4-(2-nitrophenyl)-pyridin-3,5-dicarbonsäure-(2-hydroxyethyl)-isobutyl-ester,
2,6-Dimethyl-4-(2-nitrophenyl)-pyridin-3,5-dicarbonsäure-(2-hydroxyethyl)-cyclopentyl-ester,
2,6-Dimethyl-4-(3-nitrophenyl)-pyridin-3,5-dicarbonsäure-(3-hydroxypropyl)-isopropyl-ester,
2,6-Dimethyl-4-(3-nitrophenyl)-pyridin-3,5-dicarbonsäure-(3-acetoxypropyl)-isopropyl-ester,
2,6-Dimethyl-4-(3-nitrophenyl)-pyridin-3,5-dicarbonsäure-(3-chlorpropyl)-isopropyl-ester,
2,6-Dimethyl-4-(3-nitrophenyl)-pyridin-3,5-dicarbonsäure-(3-cyanopropyl)-isopropyl-ester,
2,6-Dimethyl-4-(3-nitrophenyl)-pyridin-3,5-dicarbonsäure-(4-hydroxybutyl)-isopropyl-ester,
2,6-Dimethyl-4-(3-nitrophenyl)-pyridin-3,5-dicarbonsäure-(2-hydroxyethyl)-benzyl-ester,
2,6-Dimethyl-4-(3-nitrophenyl)-pyridin-3,5-dicarbonsäure-(2-hydroxyethyl)-(2,2,2-trifluorethyl)-ester,
2,6-Dimethyl-4-(3-nitrophenyl)-3-acetyl-pyridin-5-carbonsäure-(2-hydroxyethyl)-ester,
2,6-Dimethyl-4-(3-nitrophenyl)-3-acetyl-pyridin-5-carbonsäure-(2-acetoxyethyl)-ester,
2,6-Dimethyl-4-(3-nitrophenyl)-3-acetyl-pyridin-5-carbonsäure-(2-chlorethyl)-ester,
2,6-Dimethyl-4-(2-chlorphenyl)-pyridin-3,5-dicarbonsäure-(2-hydroxyethyl)-isopropyl-ester,
2,6-Dimethyl-4-(2,3-dichlorphenyl)-pyridin-3,5-dicarbonsäure-(2-hydroxyethyl)-isopropyl-ester,
2,6-Dimethyl-4-(2,3-dichlorphenyl)-pyridin-3,5-dicarbonsäure-(2-hydroxyethyl)-methyl-ester,
2,6-Dimethyl-4-(pyridyl-3)-pyridin-3,5-dicarbonsäure (2-hydroxyethyl)-isopropyl-ester
2,6-Dimethyl-4-(pyridyl-3)-pyridin-3,5-dicarbonsäure-(2-hydroxyethyl)-isopropyl-ester,
6-Hydroxymethyl-2-methyl-4-(3-nitrophenyl)-pyridin-3,5-dicarbonsäure-3-(2-hydroxyethyl)-5-isopropyl-ester,
6-Hydroxymethyl-2-methyl-4-(3-nitrophenyl)-pyridin-3,5-dicarbonsäure-3-(2-cyanoethyl)-5-isopropyl-ester,
5,7-Dihydro-2-methyl-4-(2-nitrophenyl)-5-oxofuro[3,4-b]-pyridin-3-carbonsäuremethylester,
5,7-Dihydro-2-methyl-4-(2-trifluormethylphenyl)-5-oxo-furo[3,4-b]pyridin-3-carbonsäureethylester,
5,7-Dihydro-2-methyl-4-(pyridyl-3)-5-oxo-furo[,4-b]-pyridin-3-carbonsäuremethylester.

Die genannten Verbindungen eignen sich besonders zur Behandlung von hypoxischen und/oder ischämischen Schädigungen vornehmlich des Zentralnervensystems sowie sklerotisch, nekrotisch oder altersbedingten cerebralen Insuffizienzen und psychopathologischen Zuständen.

Die vorteilhaften Eigenschaften seien durch folgende Untersuchungen belegt:

**a) Hypoxieschutzwirkung im Modell der Hypoxie-induzierten retrograden Amnesie im Passive Avoidance T**
(vgl. S.J. Sara & D. Lefevre, Psychopharmakologia, 25, 32-40, 1972)

In einem hell-dunkel Käfig werden Ratten mittels eines Elektroschocks trainiert, den dunklen Käfigteil zu vermeiden. Werden die Versuchstiere an-schließend einer hypoxischen Atmosphäre (3,5 Vol.-% $O_2$) ausgesetzt, wird der Gedächtnisinhalt rückwirkend zerstört. Obengenannte Verbindungen antagonisieren die retrograde Amnesie vollständig (1 mg/kg KG p.o. 30 min vor Hypoxie).

**b) Erhöhung der Hypoxietoleranz**

Substanz- und Placebo-behandelte Mäuse werden in eine Kammer gesetzt, die solange von einem hypoxischen Gasgemisch (3,5 Vol.-% $O_2$) durchströmt wird, bis 85 % der Kontrolltiere tot sind. Obengenannte Verbindungen erhöhen die Anzahl der überlebenden Tiere signifikant (10 mg/kg KG 30 min vor Hypoxie).

**c) Hemmung des Abwehrverhaltens**

Isoliert gehaltene Mäuse zeigen auf elektrische Reizung hin "aggressive-defensive behaviour". Die erfindungsgemäßen Verbindungen, die ansonsten ohne allgemein sedierende Wirkung sind, hemmen dieses Verhalten vollständig.

Die genannten Verbindungen sind stark hypoxieschutzwirksam, obwohl sie weder Blutdruck noch Herzfrequenz beeinflussen und auch am isolierten Gefäßstreifen des Kaninchens nicht vasoaktiv sind. Die gezeigte psychotrope Wirkung ist besonders in der Gerontologie von zusätzlicher therapeutischer Bedeutung.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesam-Öl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure (Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixiere, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,1 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

**Herstellungsbeispiele**

**Beispiel 1**

2,6-Dimethyl-4-(3-nitrophenyl)-pyridin-3,5-dicarbon säure-(2-hydroxyethyl)-isopropyl-ester

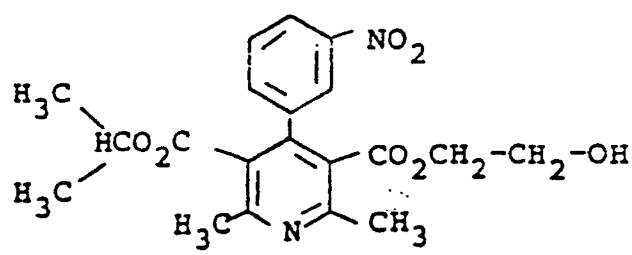

20 g (49,5 mMol) 1,4-Dihydro-2,6-dimethyl-4-(3-nitro-phenyl)-pyridin-3,5-dicarbonsäure-(2-hydroxyethyl)-isopropyl-ester wurden in eine Mischung von 83 ml 96 %iger Salpetersäure in 660 ml Wasser eingetragen und 1 Stunde zum Sieden erhitzt. Anschließend wurde auf 5° bis 10° C abgekühlt und mit verdünnter Natronlauge schwach alkalisch gestellt. Das ausgefallene Öl wurde mit Methylenchlorid extrahiert, die Extrakte über Natriumsulfat getrocknet und im Vakuum eingeengt. Der ölige Rückstand wurde durch Verreiben mit Ether/Petrolether zur Kristallisation gebracht, abgesaugt und aus Methanol umkristallisiert.

Schmelzpunkt: 120° C Ausbeute: 13,1 g (66 %)

**Beispiel 2**

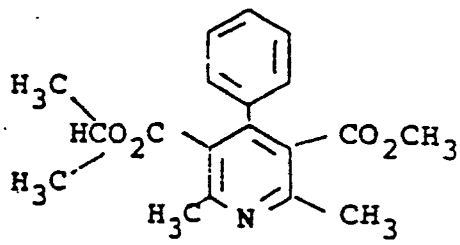

Analog Beispiel 1 wurde durch Umsetzung von 1,4-Dihydro-2,6-dimethyl-4-phenyl-pyridin-3,5-dicarbonsäure-isopropyl-methylester mit Salpetersäure der 2,6-Dimethyl-4-phenyl-pyridin-3,5-dicarbonsäure-isopropyl-methylester vom Fp: 55° C erhalten.
Ausbeute: 72 % d. Th.

**Beispiel 3**

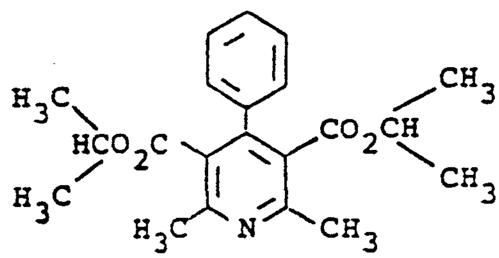

Analog Beispiel 1 wurde durch Umsetzung von 1,4-Dihydro-2,6-dimethyl-4-phenyl-pyridin-3,5-dicarbonsäurediisopropylester mit Salpetersäure der 2,6-Dimethyl-4-phenyl-pyridin-3,5-dicarbonsäurediisopropylester vom Fp. 107° C erhalten.
Ausbeute: 61 % d. Th.

**Beispiel 4**

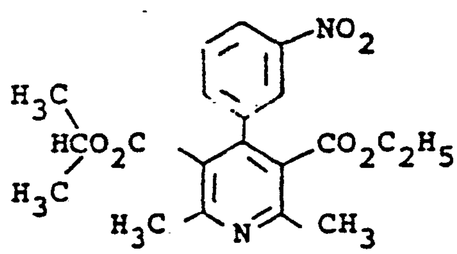

Analog Beispiel 1 wurde durch Umsetzung von 1,4-Di-hydro-2,6-dimethyl-4-(3-nitrophenyl)-pyridin-3,5-dicarbonsäure-ethyl-isopropyl-ester mit Salpetersäure der 2,6-Dimethyl-4-(3-nitrophenyl)-pyridin-3,5-dicarbonsäure-ethyl-isopropyl-ester vom Fp. 64° C erhalten.
Ausbeute: 68 % d. Th.

**Beispiel 5**

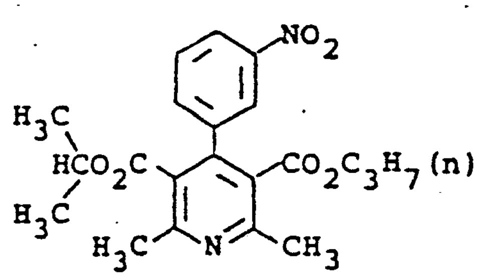

Analog Beispiel 1 wurde durch Umsetzung von 1,4-Dihydro-2,6-dimethyl-4-(3-nitrophenyl)-pyridin-3,5-dicarbonsäure-isopropyl-propylester mit Salpetersäure der 2,6-Dimethyl-4-(3-nitrophenyl)-pyridin-3,5-dicarbonsäureisopropyl-propyl-ester vom Fp. 48° C erhalten.

Ausbeute: 75 % d. Th.

**Beispiel 6**

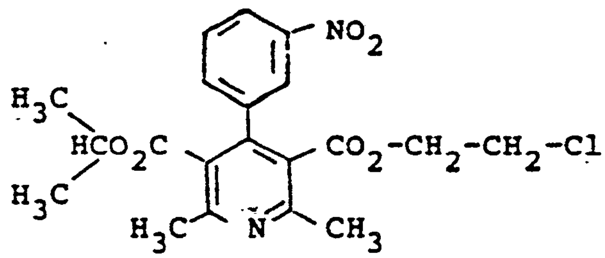

Analog Beispiel 1 wurde durch Umsetzung von 1,4-Di-hydro-2,6-dimethyl-4-(3-nitrophenyl)-pyridin-3,5-dicarbonsäure-(2-chlorethyl)-isopropyl-ester mit Salpetersäure der 2,6-Dimethyl-4-(3-nitrophenyl)-pyridin 3,5-dicarbonsäure-(2-chlorethyl)-isopropyl-ester vom Fp. 80° C erhalten.

Ausbeute: 69 % d. Th.

**Beispiel 7**

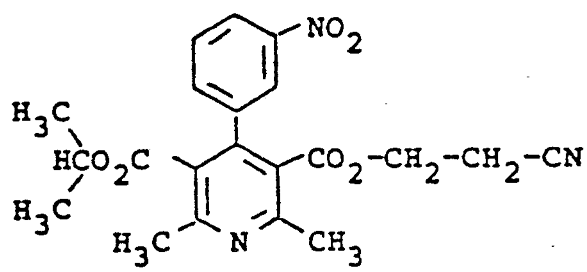

Analog Beispiel 1 wurde durch Umsetzung von 1,4-Di-hydro-2,6-dimethyl-4-(3-nitrophenyl)-pyridin-3,5-dicarbonsäure-(2-cyanoethyl)-isopropyl-ester mit Salpetersäure der 2,6-Dimethyl-4-(3-nitrophenyl)-pyridin-3,5-dicarbonsäure-(2-cyanoethyl)-isopropyl-ester vom Fp. 93° C erhalten.

Ausbeute: 71 % d. Th.

**Beispiel 8**

2,6-Dimethyl-4-(3-nitrophenyl)-pyridin-3,5-dicarbonsäure-(2-acetoxyethyl)-isopropyl-ester

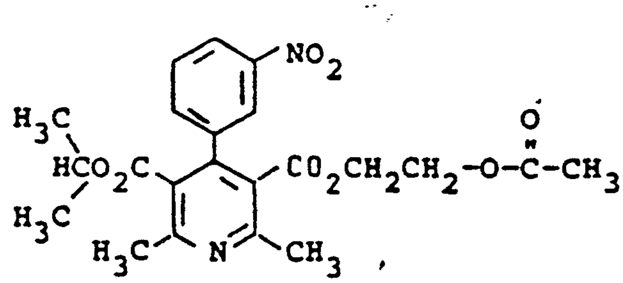

20,1 g (50 mMol) 2,6-Dimethyl-4-(3-nitrophenyl)-pyridin-3,5-dicarbonsäure-(2-acetoxyethyl)-isopropyl-ester (Beispiel 1) wurden in 75 ml Pyridin gelöst. Dazu wurden 5,9 g (75 mMol) Acetylchlorid zugegeben. Nach Beendigung der exothermen Reaktion wurde 3 Stunden bei Raumtemperatur gerührt, das Reaktionsgemisch in Wasser gegossen und mit $CH_2Cl_2$ extrahiert. Die organischen Extrakte wurden mit verdünnter Chlorwasserstoffsäure gewaschen und nach dem Trocknen über Natriumsulfat im Vakuum eingeengt. Das resultierende Öl kristallisierte durch, es wurde mit Petrolether verrührt, abgesaugt und getrocknet. Fp. 68°C, Ausbeute: 20,5 g (93 %)

**Beispiel 9**

2,6-Dimethyl-4-(3-nitrophenyl)-pyridin-3,5-dicarbonsäure-isopropyl-(2-methoxyethyl)-ester

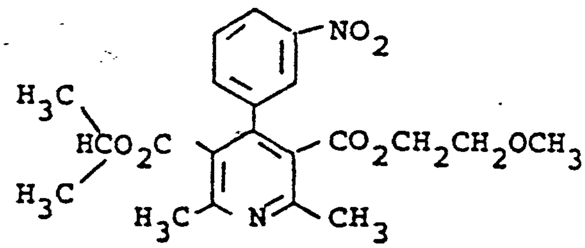

Zu einer siedenden Lösung von 41,8 g (100 mMol) 1,4-Di-hydro-2,6-dimethyl-4-(3-nitrophenyl)-pyridin-3,5-dicarbonsäure-isopropyl-(2-methoxyethyl)-ester in 160 ml Eisessig wurden portionsweise 6,8 g Chrom(VI)oxid zugegeben. Anschließend wurde weitere 30 Minuten unter Rückfluß erhitzt und nach dem Abkühlen in ammoniakalisches Eiswasser gegossen. Die Mischung wurde mit Chloroform extrahiert und die Extrakte nach dem Trocknen über Natriumsulfat im Vakuum eingeengt. Es resultierten 35,9 g (86 % d. Th.) eines Öls.

**Beispiel 10**

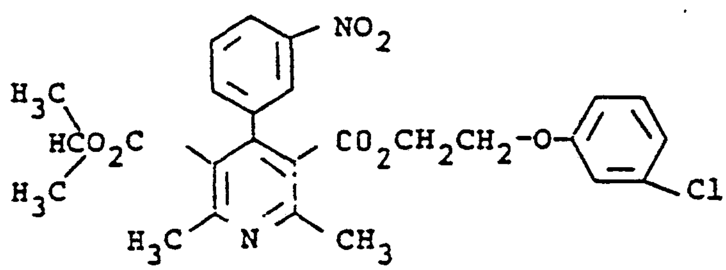

Analog Beispiel 1 wurde durch Umsetzung von 1 4-Dihydro-2,6-dimethyl-4-(3-nitrophenyl)-pyridin-3,5-dicarbonsäure-(2-(3-chlorphenoxy)-ethyl)-isopropyl-ester mit Salpetersäure der 2,6-Dimethyl-4-(3-nitrophenyl)-pyridin-3,5-dicarbonsäure-(2-(3-chlorphenoxy)-ethyl)-isopropyl-ester vom Fp. 79°C erhalten.

Ausbeute: 85 % d. Th.

**Beispiel 11**

2,6-Dimethyl-4-(3-nitrophenyl)-pyridin-3,5-dicarbonsäuremonoisopropylester

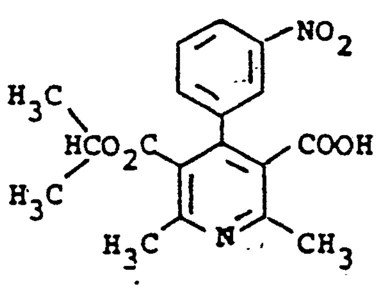

1 g (2,5 mMol) 2,6-Dimethyl-4-(3-nitrophenyl)-pyridin-3,5-dicarbonsäurediisopropylester wurde in einer Lösung von 140 mg (2,5 mMol) Kaliumhydroxid in 10 ml Ethanol und 1 ml Wasser 5 Stunden unter Rückfluß erhitzt. Nach dem Abkühlen wurde zur Trockne eingeengt, der Rückstand in Wasser aufgenommen und mehrmals mit Chloroform extrahiert. Die wäßrige Phase wurde mit verdünnter Chlorwasserstoffsäure auf pH 3 bis 4 gestellt, das ausgefallene Produkt abgesaugt und erneut mit gesättigter Natriumbicarbonatlösung in Lösung gebracht. Nach Zugabe von etwas Tierkohle wurde filtriert und das Filtrat mit verdünnter Chlorwasserstoffsäure auf pH 3 bis 4 gestellt. Das ausgefallene Produkt wurde abgesaugt und im Vakuum getrocknet. Fp: 234-236°C. Ausbeute: 300 mg (33 % d. Th.)

**Beispiel 12**

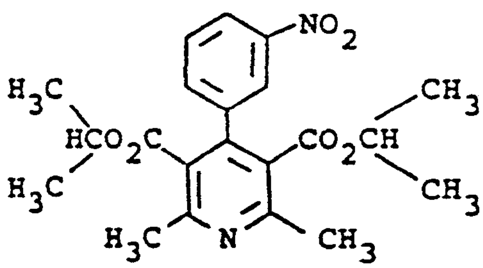

Analog Beispiel 1 wurde durch Umsetzung von 1,4-Di-hydro-2,6-dimethyl-4-(3-nitrophenyl)-pyridin-3,5-dicarbonsäurediisopropylester mit Salpetersäure der 2,6-Dimethyl-4-(3-nitrophenyl)-pyridin-3,5-dicarbonsäurediisopropylester vom Fp. 77° C erhalten.
Ausbeute: 66 % d.Th.

**Beispiel 13**

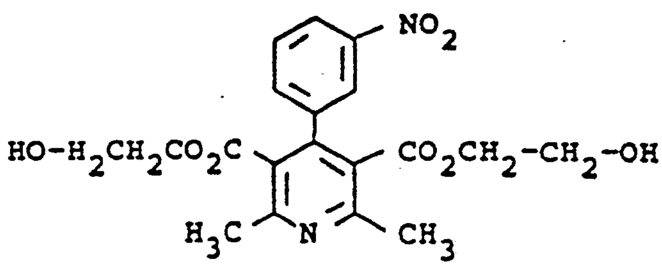

Analog Beispiel 1 wurde durch Umsetzung von 1,4-Di-hydro-2,6-dimethyl-4-(3-nitrophenyl)-pyridin-3 5-dicarbonsaäure-bis-(2-hydroxyethyl)-ester mit Salipetersäure der 2,6-Dimethyl-4-(3-nitrophenyl)-pyridin-3 5-dicarbonsäure-bis-(2-hydroxyethyl)-ester vom Fp. 121° C erhalten.
Ausbeute: 81 % d. Th.

**Beispiel 14**

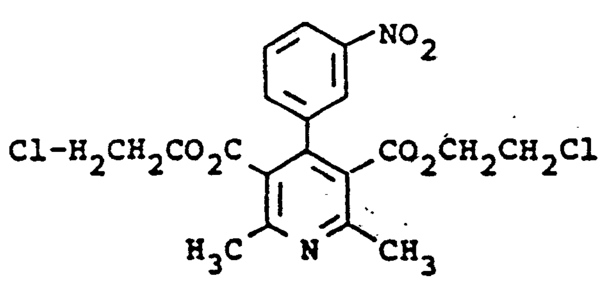

Analog Beispiel 1 wurde durch Umsetzung von 1,4-Di-hydro-2,6-dimethyl-4-(3-nitrophenyl)-pyridin-3,5-dicarbonsäure-bis-(2-chlorethyl)-ester mit Salpetersäure der 2,6-Dimethyl-4-(3-nitrophenyl)-pyridin-3,5-dicarbonsäure-bis-(2-chlorethyl)-ester vom Fp. 72° C erhalten.
Ausbeute: 80 % d. Th.

**Beispiel 15**

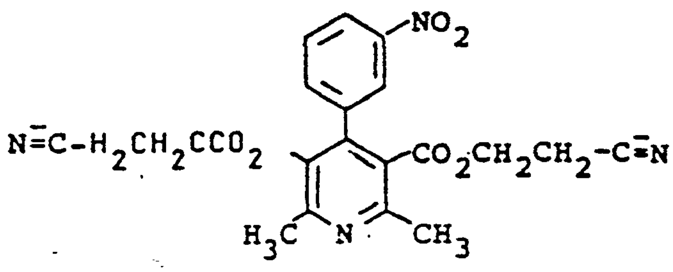

Analog Beispiel 1 wurde durch Umsetzung von 1,4-Di-hydro-2 6-dimethyl-4-(3-nitrophenyl)-pyridin-3,5-dicarbonsäure-bis-(2-cyanoethyl)-ester mit Salpetersäure der 2,6-Dimethyl-4-(3-nitrophenyl)-pyridin-3,5-dicarbonsäure-bis-(2-cyanoethyl)-ester vom Fp. 130° C erhalten.
Ausbeute: 65 % d. Th.

**Beispiel 16**

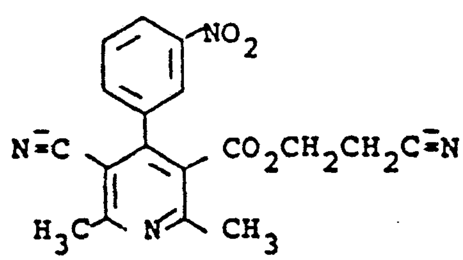

Analog Beispiel 1 wurde durch Umsetzung von 3-Cyano-1,4-dihydro-2,6-dimethyl-4-(3-nitrophenyl)-pyridin-5-carbonsäure-(2-cyanoethyl)-ester mit Salpetersäure der 3-Cyano-2,6-dimethyl-4-(3-nitrophenyl)-pyridin-5-carbonsäure-(2-cyanoethyl)-ester vom Fp. 142°C erhalten.
Ausbeute: 69 % d. Th.

**Beispiel 17**

3-Acetyl-2,6-dimethyl-4-(3-nitrophenyl)-pyridin-5-car-bonsäure-(2-cyanoethyl)-ester

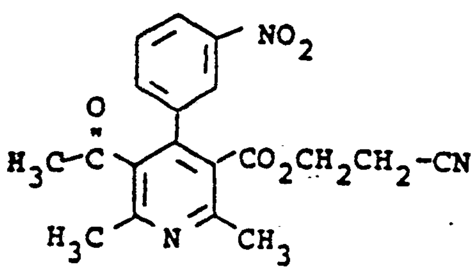

Eine Lösung von 10 g (27 mMol) 3-Acetyl-1,4-dihydro-2,6-dimethyl-4-(3-nitrophenyl)-pyridin-5-carbonsäure-(2-cyanoethyl)-ester in einem Elektrolyten von 5 g Lithiumperchlorat in 250 ml Acetonitril wurde an einer Platin-Anode bei einem Elektrodenpotential von + 1.2 V vs. SCE elektrolysiert. Nach Durchgang von 2 Faraday-Äquivalenten wurde die Elektrolyse beendet, der Anolyt im Vakuum eingeengt, der Rückstand in einer Natriumbicarbonatlösung aufgenommen und mehrmals mit Methylenchlorid extrahiert. Die organischen Extrakte wurden nach Waschen mit Wasser über Natriumsulfat getrocknet und anschließend im Vakuum eingeengt. Das resultierende Öl kristallisierte durch, das feste Produkt wurde in Petrolether verrührt, abgesaugt und getrocknet, Fp. 85° C, Ausbeute: 7,5 g (75 %)

**Beispiel 18**

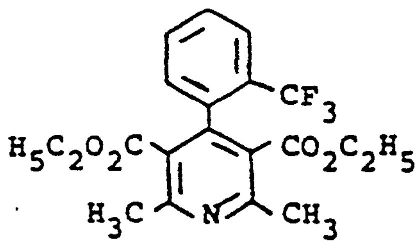

Analog Beispiel 17 wurde durch anodische Oxidation des 1,4-Dihydro-2,6-dimethyl-4-(2-trifluormethylphenyl)-pyridin-3,5-dicarbonsäurediethylester in Acetonitril/ $LiClO_4$ der 2,6-Dimethyl-4-(2-trifluormethylphenyl)-pyridin-3,5-dicarbonsäurediethylester vom Fp. 92° C erhalten.

Ausbeute: 83 % d. Th.

**Beispiel 19**

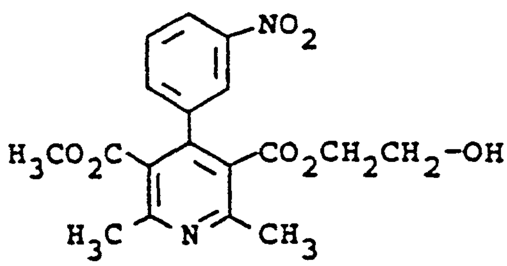

Analog Beispiel 1 wurde durch Umsetzung von 1,4-Dihydro-2,6-dimethyl-4-(3-nitrophenyl)-pyridin-3,5-dicarbonsäure-(2-hydroxyethyl)-methyl-ester mit Salpetersäure der 2,6-Dimethyl-4-(3-nitrophenyl)-pyridin-3,5-dicarbonsäure-(2-hydroxyethyl)-methyl-ester vom Fp. 115° C erhalten.

Ausbeute 59 % d. Th.

**Beispiel 20**

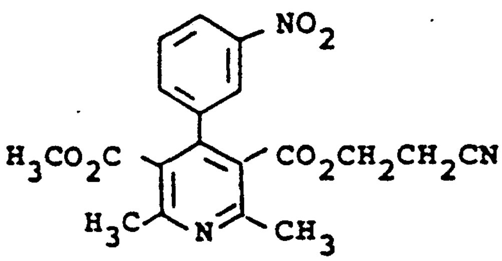

Analog Beispiel 1 wurde durch Umsetzung von 1,4-Dihydro-2,6-dimethyl-4-(3-nitrophenyl)-pyridin-3,5-dicarbon-säure-(2-cyanoethyl)-methyl-ester mit Salpetersäure der 2,6-Dimethyl-4-(3-nitrophenyl)-pyridin-3,5-dicarbonsäure-(2-cyanoethyl)-methyl-ester vom Fp. 98° C erhalten.

Ausbeute 65 % d. Th.

**Beispiel 21**

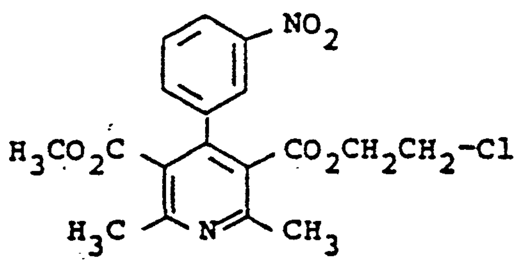

Analog Beispiel 1 wurde durch Umsetzung von 1,4-Dihydro-2,6-dimethyl-4-(3-nitrophenyl)-pyridin-3,5-dicarbonsäure-(2-chlorethyl)-methyl-ester mit Salpetersäure der 2,6-Dimethyl-4-(3-nitrophenyl)-pyridin-3,5-dicarbonsäure-(2-chlorethyl)-methylester vom Fp. 60° C erhalten. Ausbeute 55 % d. Th.

**Beispiel 22**

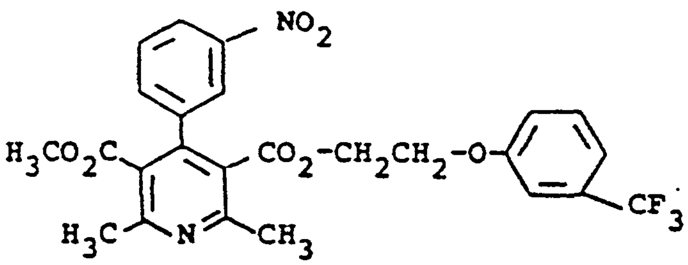

Analog Beispiel 1 wurde durch Umsetzung von 1,4-Dihydro-2,6-dimethyl-4-(3-nitrophenyl)-pyridin-3,5-dicarbonsäure-methyl-(2-(3-trifluormethylphenoxy)-ethyl)-ester mit Salpetersäure der 2,6-Dimethyl-4-(3-nitrophenyl)-pyridin-3,5-dicarbonsäure-methyl-(2-(3-trifluormethylphenoxy)-ethyl)-ester vom Fp. 88° C erhalten. Ausbeute 89 % d. Th.

**Beispiel 23**

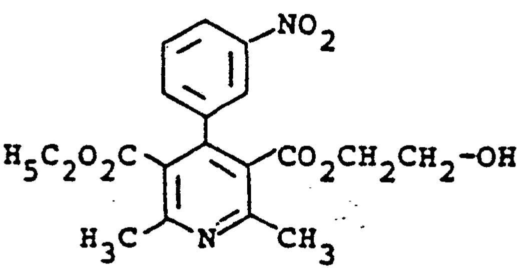

Analog Beispiel 1 wurde durch Umsetzung von 1,4-Dihydro-2,6-dimethyl-4-(3-nitrophenyl)-pyridin-3,5-dicarbonsäure-ethyl-(2-hydroxyethyl)-ester mit Salpetersäure der 2,6-Dimethyl-4-(3-nitrophenyl)-pyridin-3,5-dicarbonsäure-ethyl-(2-hydroxyethyl)-ester vom Fp. 88° C erhalten.
Ausbeute 51 % d Th

**Beispiel 24**

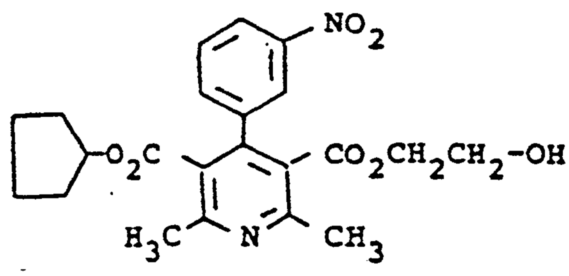

Analog Beispiel 1 wurde durch Umsetzung von 1,4-Dihydro-2,6-dimethyl-4-(3-nitrophenyl)-pyridin-3,5-dicarbonsäure-cyclopentyl-(2-hydroxyethyl)-ester mit Salpetersäure der 2,6-Dimethyl-4-(3-nitrophenyl)-pyridin-3,5-dicarbonsäure-cyclopentyl-(2-hydroxyethyl)-ester vom Fp. 112° C erhalten.

Ausbeute: 63 % d. Th.

**Beispiel 25**

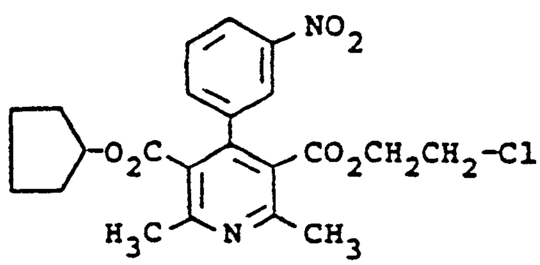

Analog Beispiel 1 wurde durch Umsetzung von 1,4-Dihydro-2,6-dimethyl-4-(3-nitrophenyl)-pyridin-3,5-dicarbonsäure-(2-chlorethyl)-cyclopentyl-ester mit Salpetersaure der 2,6-Dimethyl-4-(3-nitrophenyl)-pyridin-3,5-dicarbonsäure-(2-chlorethyl)-cyclopentyl-ester vom Fp. 84° C erhalten.

Ausbeute 71 % d. Th.

**Beispiel 26**

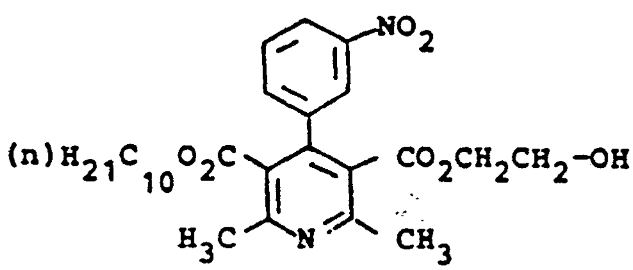

Analog Beispiel 1 wurde durch Umsetzung von 1,4-Dihydro-2,6-dimethyl-4-(3-nitrophenyl)-pyridin-3,5-dicarbonsäure-decyl-(2-hydroxyethyl)-ester mit Salpetersäure der 2,6-Dimethyl-4-(3-nitrophenyl)-pyridin-3,5-dicarbonsäure-decyl-(2-hydroxyethyl)-ester vom Fp. 60° C erhalten.

Ausbeute 71 % d. Th.

**Beispiel 27**

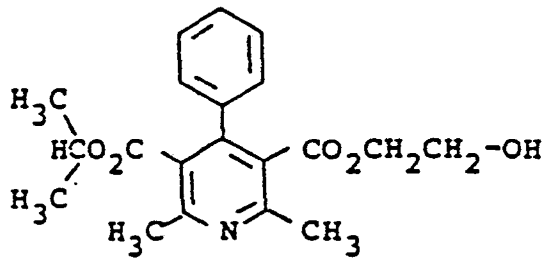

Analog Beispiel 1 wurde durch Umsetzung von 1,4-Dihydro-2,6-dimethyl-4-phenyl-pyridin-3,5-dicarbonsäure-(2-hydroxyethyl)-isopropylester mit Salpetersäure der 2,6-Di-methyl-4-phenyl-pyridin-3,5-dicarbonsäure-(2-hydroxyethyl)-isopropyl-ester vom Fp. 79° C erhalten.
Ausbeute 82 % d. Th.

**Beispiel 28**

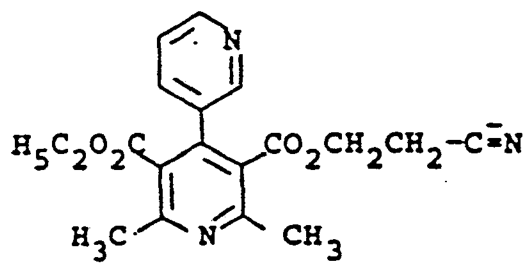

Analog Beispiel 1 wurde durch Umsetzung von 1,4-Dihydro 2,6-dimethyl-4-(pyridyl-3)-pyridin-3,5-dicarbonsäure-(2-cyanoethyl)-ethyl-ester mit Salpetersäure der 2,6-Dimethyl-4-(pyridyl-3)-pyridin-3,5-dicarbonsäure-(2-cyanoethyl)-ethyl-ester vom Fp. 90° C erhalten.
Ausbeute 52 % d. Th.

**Beispiel 29**

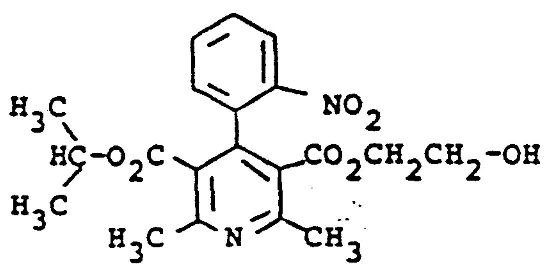

Analog Beispiel 1 wurde durch Umsetzung von 1,4-Dihydro-2,6-dimethyl-4-(2-nitrophenyl)-pyridin-3,5-dicarbonsäure-(2-hydroxyethyl)-isopropyl-ester mit Salpetersäure der 2,6-Dimethyl-4-(2-nitrophenyl)-pyridin-3,5-dicarbonsäure-(2-hydroxyethyl)-isopropyl-ester vom Fp. 78° C erhalten.
Ausbeute 83 %

**Beispiel 30**

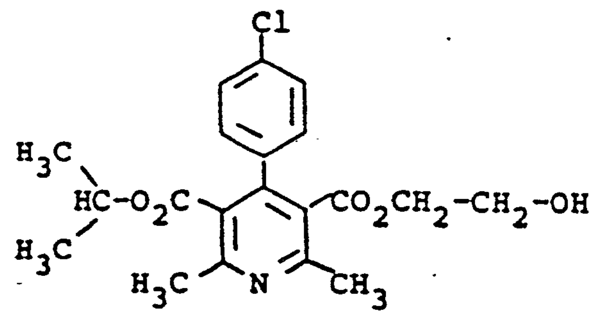

Analog Beispiel 1 wurde durch Umsetzung von 1,4-Dihydro-2,6-dimethyl-4-(4-chlorphenyl)-pyridin-3,5-dicarbonsäure-(2-hydroxyethyl)-isopropyl-ester mit Salpetersäure der 2,6-Dimethyl-4-(4-chlorphenyl)-pyridin-3,5-dicarbonsäure-(2-hydroxyethyl)-isopropyl-ester vom Fp. 120° C erhalten.
Ausbeute 88 % d. Th.

**Beispiel 31**

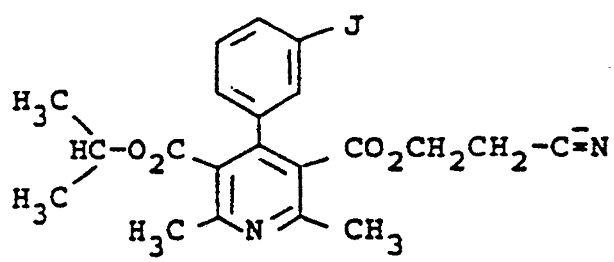

Analog Beispiel 1 wurde durch Umsetzung von 1,4-Dihydro-2,6-dimethyl-4-(3-jodphenyl)-pyridin-3,5-dicarbonsäure-(2-cyanoethyl)-isopropyl-ester mit Salpetersäure der 2,6-Dimethyl-4-(3-jodphenyl)-pyridin-3,5-dicarbonsäure-(2-cyanoethyl)-isopropyl-ester vom Fp. 69° C erhalten.
Ausbeute 75 % d. Th.

**Beispiel 32**

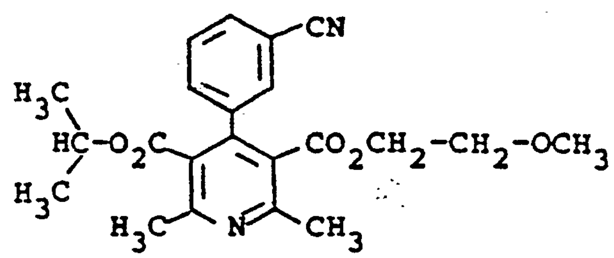

Analog Beispiel 1 wurde durch Umsetzung von 1,4-Dihydro-2,6-Dimethyl-4-(3-cyanophenyl)-pyridin-3,5-dicarbonsäure isopropyl-(2-methoxyethyl)-ester mit Salpetersäure der 2,6-Dimethyl-4-(3-cyanophenyl)-pyridin-3,5-dicarbonsäure-isopropyl-(2-methoxyethyl)-ester vom Fp. 50° C erhalten.
Ausbeute 73 % d. Th.

**Beispiel 33**

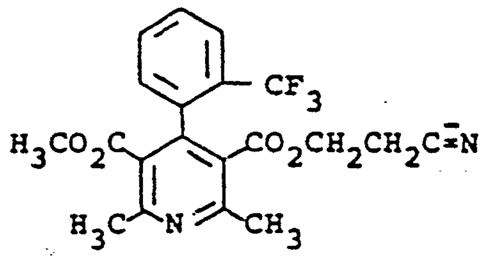

Analog Beispiel 1 wurde durch Umsetzung von 1,4-Dihydro-2,6-dimethyl-4-(2-trifluormethylphenyl)-pyridin-3,5-dicarbonsäure-(2-cyanoethyl)-methylester mit Salpetersäure der 2,6-Dimethyl-4-(2-trifluormethylphenyl)-pyridin-3,5-dicarbonsäure-(2-cyanoethyl)-methylester vom Fp. 74° C erhalten.
Ausbeute 85 % d. Th.

**Beispiel 34**

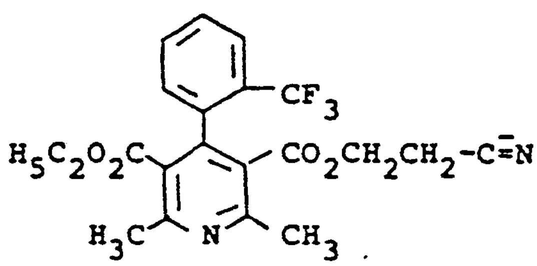

Analog Beispiel 1 wurde durch Umsetzung von 1,4-Dihydro-2,6-dimethyl-4-(2-trifluormethylphenyl)-pyridin-3,5-dicarbonsäure-(2-cyanoethyl)-ethyl-ester mit Salpetersäure der 2,6-Dimethyl-4-(2-trifluormethylphenyl)-pyridin-3,5-dicarbonsäure-(2-cyanoethyl)-ethyl-ester vom Fp. 68° C erhalten.
Ausbeute 69 % d. Th.

**Beispiel 35**

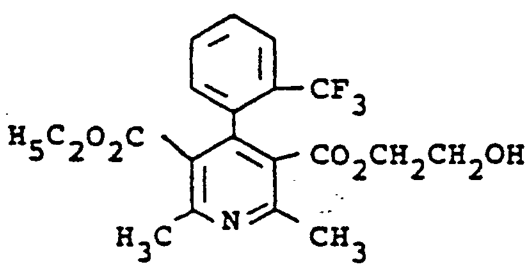

Analog Beispiel 1 wurde durch Umsetzung von 1,4-Dihydro-2,6-dimethyl-4-(2-trifluormethylphenyl)-pyridin-3,5-dicarbonsäure-ethyl-(2-hydroxyethyl)-ester mit Salpetersäure der 2,6-Dimethyl-4-(2-trifluormethylphenyl)-pyridin-3,5-dicarbonsäure-ethyl-(2-hydroxyethyl)-ester vom Fp. 86° C erhalten.
Ausbeute 75 % d. Th.

**Beispiel 36**

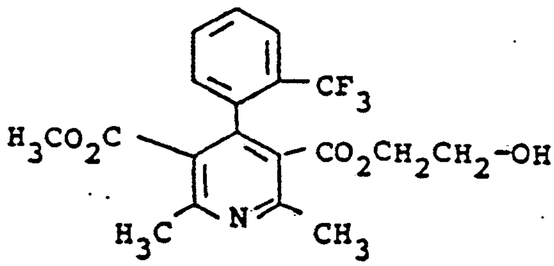

Analog Beispiel 1 wurde durch Umsetzung von 1,4-Dihydro-2,6-dimethyl-4-(2-trifluormethylphenyl)-pyridin-3,5-dicarbonsäure-(2-hydroxyethyl)-methyl-ester mit Salpetersäure der 2,6-Dimethyl-4-(2-trifluormethylphenyl)-pyridin-3,5-dicarbonsäure-(2-hydroxyethyl)-methyl-ester vom Fp. 75°C erhalten.

Ausbeute 71 % d. Th.

**Beispiel 37**

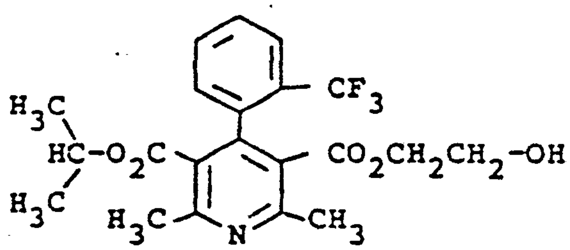

Analog Beispiel 1 wurde durch Umsetzung von 1,4-Dihydro-2,6-dimethy1-4-(2-trifluormethylphenyl)-pyridin-3,5-dicarbonsäure-(2-hydroxyethyl)-isopropopyl-ester mit Salpetersäure der 2,6-Dimethyl-4-(2-trifluormethylphenyl)-pyridin-3,5-dicarbonsäure-(2-hydroxyethyl)-isopropyl-15 ester vom Fp. 83°C erhalten.

Ausbeute 51 % d. Th.

**Beispiel 38**

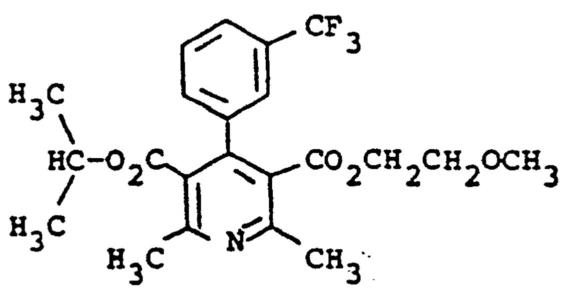

Analog Beispiel 1 wurde durch Umsetzung von 1,4-Dihydro-2,6-dimethyl-4-(3-trifluormethylphenyl)-pyridin-3,5-dicarbonsäure-isopropyl-(2-methoxyethyl)-ester mit Salpetersäure der 2,6-Dimethyl-4-(3-trifluormethylphenyl)-pyridin-3,5-dicarbonsäure-isopropyl-(2-methoxyethyl)-ester vom Fp. 51°C erhalten.

Ausbeute 65 % d. Th.

**Beispiel 39**

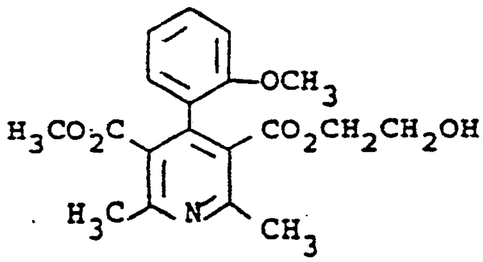

Analog Beispiel 1 wurde durch Umsetzung von 1,4-Dihydro-2,6-dimethyl-4-(2-methoxyphenyl)-pyridin-3,5-dicarbonsäure-(2-hydroxyethyl)-methyl-ester mit Salpetersäure der 2,6-Dimethyl-4-(2-methoxyphenyl)-pyridin-3,5-di-carbonsäure-(2-hydroxyethyl)-methyl-ester vom Fp. 90° C erhalten.
Ausbeute 84 % d. Th.

**Beispiel 40**

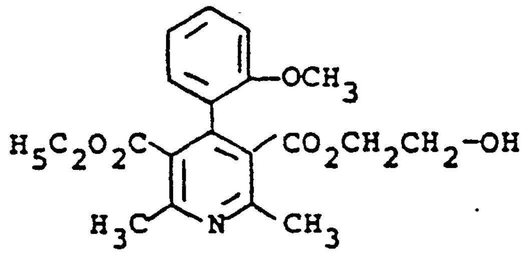

Analog Beispiel 1 wurde durch Umsetzung von 1,4-Dihydro-2,6-dimethyl-4-(2-methoxyphenyl)-pyridin-3,5-dicarbonsäure-ethyl-(2-hydroxyethyl)-ester mit Salpetersäure der 2,6-Dimethyl-4-(2-methoxyphenyl)-pyridin-3,5-dicarbonsäure-ethyl-(2-hydroxyethyl)-ester vom Fp. 95° C erhalten.
Ausbeute 71 % d. Th.

**Beispiel 41**

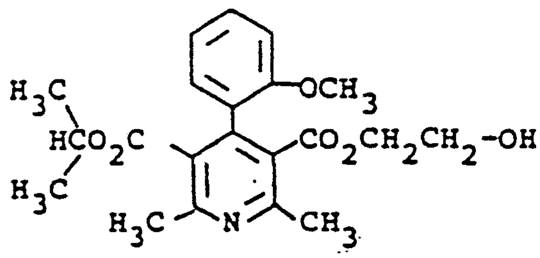

Analog Beispiel 1 wurde durch Umsetzung von 1,4-Dihydro-2 6-dimethyl-4-(2-methoxyphenyl)-pyridin-3,5-dicarbonsäure-(2-hydroxyethyl)-isopropyl-ester mit Salpetersäure der 2,6-Dimethyl-4-(2-methoxyphenyl)-pyridin-3,5-dicarbonsäure-(2-hydroxyethyl)-isopropyl-ester vom Fp. 68° C erhalten.
Ausbeute 85 % d. Th.

**Beispiel 42**

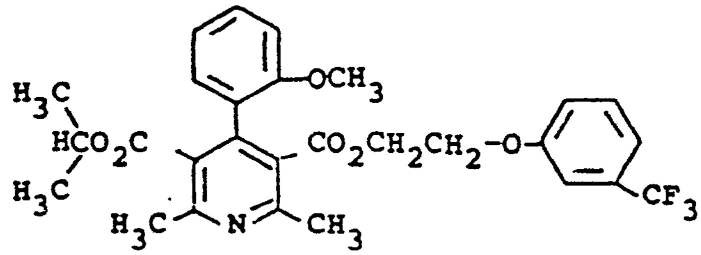

Analog Beispiel 1 wurde durch Umsetzung von 1,4-Dihydro-2,6-dimethyl-4-(2-methoxyphenyl)-pyridin-3,5-dicarbonsäure-isopropyl-(2-(3-trifluormethylphenoxy)-ethyl)-ester mit Salpetersäure der 2,6-Dimethyl-4-(2-methoxy-phenyl)-pyridin-3,5-dicarbonsäure-isopropyl-(2-(3-tri fluormethylphenoxy)-ethyl)-ester vom Fp. 80°C erhalten.
Ausbeute 65 % d. Th.

**Beispiel 43**

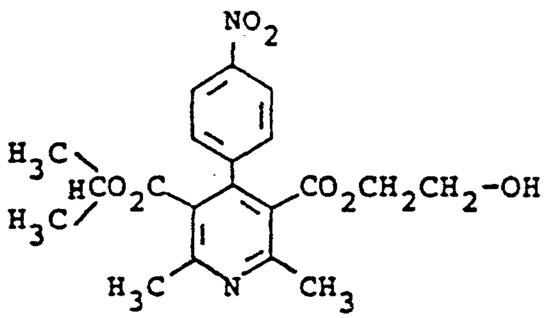

Analog Beispiel 1 wurde durch Umsetzung von 1,4-Dihydro-2,6-dimethyl-4-(4-nitrophenyl)-pyridin-3,5-dicarbonsäure-(2-hydroxyethyl)-isopropyl-ester mit Salpetersäure der 2,6-Dimethyl-4-(4-nitrophenyl)-pyridin-3,5-dicarbonsäure-(2-hydroxyethyl)-isopropyl-ester vom Fp. 145°C erhalten.
Ausbeute 62 % d. Th.

**Beispiel 44**

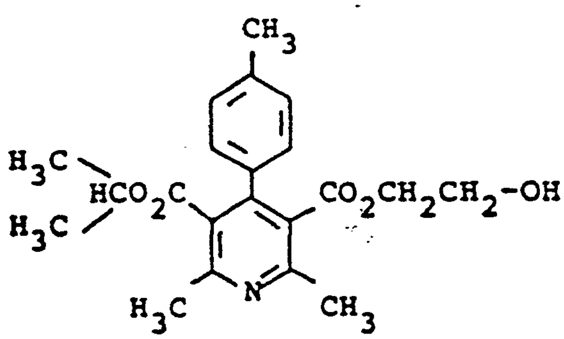

Analog Beispiel 1 wurde durch Umsetzung von 1,4-Dihydro-2,6-dimethyl-4-(4-methylphenyl)-pyridin-3,5-dicarbonsäure-(2-hydroxyethyl)-isopropyl-ester mit Salpetersäure der 2,6-Dimethyl-4-(4-methylphenyl)-pyridin-3,5-dicarbonsäure-(2-hydroxyethyl)-isopropyl-ester vom Fp. 72°C erhalten.
Ausbeute 56 % d. Th.

**Beispiel 45**

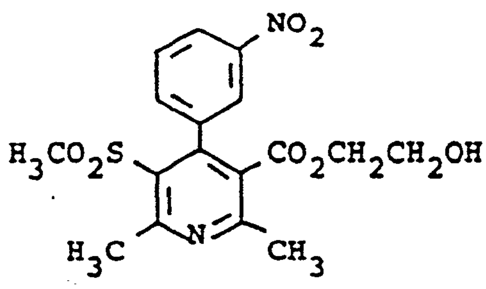

Analog Beispiel 1 wurde durch Umsetzung von 1,4-Dihydro-2,6-dimethyl-3-methylsulfonyl-4-(3-nitrophenyl)-pyridin-5-carbonsäure-(2-hydroxyethyl)-ester mit Salpetersäure der 2,6-Dimethyl-3-methylsulfonyl-4-(3-nitrophenyl)-pyridin-5-carbonsäure-(2-hydroxyethyl)-ester vom Fp. 120°C erhalten.
Ausbeute 53 % d. Th.

**Beispiel 46**

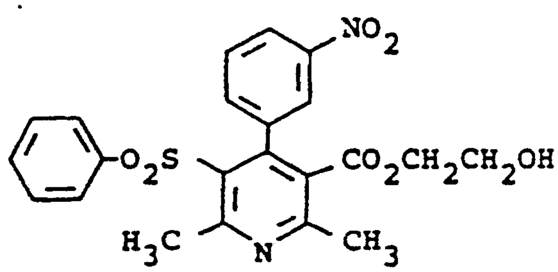

Analog Beispiel 1 wurde durch Umsetzung von 1,4-Dihydro-2,6-dimethyl-4-(3-nitrophenyl)-3-phenylsulfonyl-pyridin-5-carbonsäure-(2-hydroxyethyl)-ester mit Salpetersäure der 2,6-Dimethyl-4-(3-nitrophenyl)-3-phenylsulfonyl-pyridin-5-carbonsäure-(2-hydroxyethyl)-ester vom Fp. 150°C erhalten.
Ausbeute 41 % d. Th.

**Beispiel 47**

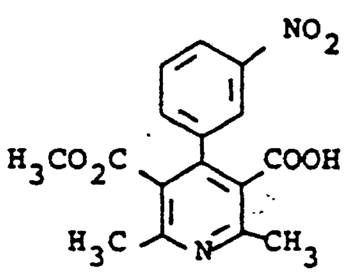

Analog Beispiel 11 wurde durch Umsetzung von 2,6-Di-methyl-4-(3-nitrophenyl)-pyridin-3,5-dicarbonsäure-dimethylester mit methanolischer Kalilauge der 2,6-Dimethyl-4-(3-nitrophenyl)-pyridin-3,5-dicarbonsäure-monomethylester vom Fp. 202°C erhalten.
Ausbeute 65 % d. Th.

**Beispiel 48**

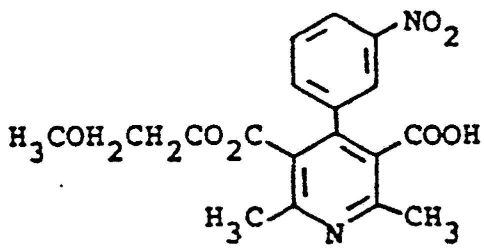

Analog Beispiel 11 wurde durch Umsetzung von 2,6-Di-methyl-4-(3-nitrophenyl)-pyridin-3,5-dicarbonsäure-bis-(2-methoxyethyl)-ester mit methoxyethanolischer Kalilauge der 2,6-Dimethyl-4-(3-nitrophenyl)-pyridin-3,5-dicarbonsäuremono-(2-methoxyethyl)-ester vom Fp. 191°C erhalten.

Ausbeute 58 % d. Th.

**Beispiel 49**

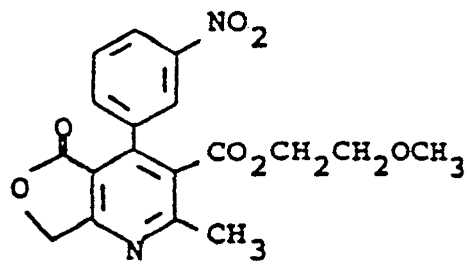

Analog Beispiel 9 wurde durch Umsetzung von 1,4,5,7-Tetrahydro-2-methyl-4-(3-nitrophenyl)-5-oxo-furo-[3,4-b]-pyridin-3-carbonsäure-(2-methoxyethyl)-ester mit Chrom(VI)oxid in Eisessig der 5,7-Dihydro-2-methyl-4-(3-nitrophenyl)-5-oxo-furo[3,4-b]pyridin-3-carbonsäure-(2-methoxyethyl)-ester vom Fp. 114°C erhalten.

Ausbeute 75 % d. Th.

**Beispiel 50**

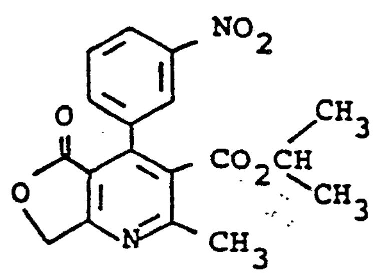

Analog Beispiel 9 wurde durch Umsetzung von 1,4,5,7-Tetrahydro-2-methyl-4-(3-nitrophenyl)-5-oxo-furo-[3,4-b]pyridin-3-carbonsäureisopropylester mit Chrom(VI)oxid in Eisessig der 5,7-Dihydro-2-methyl-4-(3-nitrophenyl)-5-oxo-furo[3,4-b]pyridin-3-carbon-säureisopropyl-ester vom Fp. 181°C erhalten.

Ausbeute 80 % d. Th.

**Beispiel 51**

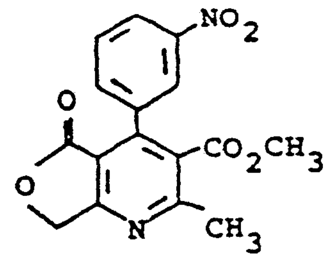

Analog Beispiel 9 wurde durch Umsetzung von 1,4,5,7-Tetrahydro-2-methyl-4-(3-nitrophenyl)-5-oxo-furo [3,4-b]pyridin-3-carbonsäuremethylester mit Chrom(VI)oxid in Eisessig der 5,7-Dihydro-2-methyl-4-(3-nitro-phenyl)-5-oxo-furo[3,4-b]pyridin-3-carbonsäuremethylester vom Fp. 214°C erhalten.
Ausbeute: 78 % d. Th.

**Beispiel 52**

Kalium-Salz des 2-Hydroxymethyl-6-methyl-4-(3-nitro-phenyl)-pyridin-3,5-dicarbonsäure-5-(2-hydroxyethyl)-esters

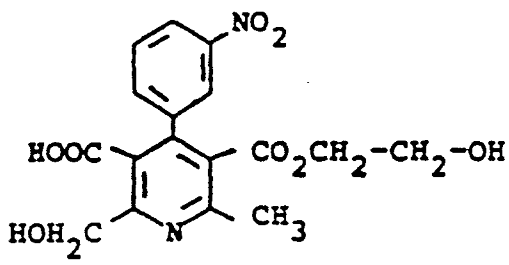

24 g (50 mMol) 2-Acetoxymethyl-6-methyl-4-(3-nitro-phenyl)-pyridin-3,5-dicarbonsäure-5-(2-acetoxyethy1)-3-ethyl-ester wurden in 120 ml 1 2-Dimethoxyethan gelöst und nach vorsichtiger Zugabe einer Lösung von 6,2 g Kaliumhydroxid in 120 ml Wasser zwei Stunden bei Raumtemperatur gerührt. Anschließend wurde die Mischung mehrmals mit Methylenchlorid extrahiert, die wäßrige Phase im Vakuum zur Trockne eingedampft und der Rückstand aus Isopropanol umkristallisiert. Fp. 223°C. Ausbeute 3,6 g (17 % d. Th.)

**Beispiel 53**

5,7-Dihydro-2-methyl-4-(3-nitrophenyl)-5-oxo-furo[3,4-b]-1)-ester

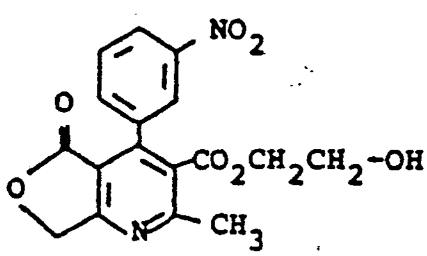

4 g des Kalium-Salzes des 2-Hydroxymethyl-6-methyl-4-(3-nitrophenyl)-pyridin-3,5-dicarbonsäure-5-(2-hydroxyethyl)-esters (Beispiel 52) wurden in 4 ml Wasser gelöst und mit konzentrierter Chlorwasserstoffsäure angesäuert. Nach Stehen über Nacht bei Raumtemperatur wurde mit Wasser verdünnt, das ausgefallene Öl mit Methylenchlorid extrahiert, die organischen Extrakte nach Trocknen über Natriumsulfat im Vakuum eingeengt und der Rückstand aus Isopropanol umkristallisiert, Fp. 130°C. Ausbeute: 2,3 g (64 % d. Th.)

**Patentansprüche**

1. Verwendung von Verbindungen der allgemeinen Formel I

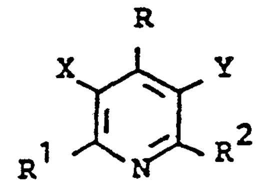

(I)

in welcher
R für Phenyl oder Pyridyl steht, wobei der Phenylrest gegebenenfalls 1- oder 2-fach substituiert ist durch Nitro, Cyano, Trifluormethyl, Fluor, Chlor, Brom, Jod, Alkyl, Alkoxy, Alkylmercapto mit jeweils 1 bis 2 C-Atomen in den Alkyl- und Alkoxyresten,
$R^1$ und $R^2$ gleich oder verschieden sind und jeweils für Wasserstoff oder einen Alkylrest mit 1 bis 4 Kohlenstoffatomen stehen, der gegebenenfalls durch Hydroxy substituiert ist,
oder
$R^1$ zusammen mit X oder $R^2$ zusammen mit Y gemeinsam einen 5- bis 7-gliedrigen Ring bilden, der durch Carbonyl und gegebenenfalls durch Sauerstoff, und/ oder NH unterbrochen ist und
X und Y gleich oder verschieden sind und jeweils für eine Nitrilgruppe stehen
oder
für den Rest $-COR^3$ stehen, wobei $R^3$ einen Alkylrest mit 1 bis 4 Kohlenstoffatomen, Phenyl oder Benzyl bedeutet
oder
für die Gruppe $-COOR^4$ stehen, wobei $R^4$ Wasserstoff, für einen geradkettigen, verzweigten oder cyclischen gesättigten oder ungesättigten Kohlenwasserstoffrest mit bis zu 10 Kohlenstoffatomen bedeutet, der gegebenenfalls durch 1 Sauerstoffatom in der Kette unterbrochen ist und/oder der gegebenenfalls substituiert ist durch Hydroxy, Fluor, Chlor, Brom, Cyano, Acetoxy, Pyridyl, Phenyl oder Phenoxy, wobei die Phenylgruppen ihrerseits gegebenenfalls durch Fluor, Chlor, Trifluormethyl, Nitro, Alkyl mit 1 bis 4 C-Atomen oder Alkoxy mit 1 bis 4 C-Atomen substituiert sein können
oder
für die Gruppe $-SO_2R^5$ stehen, wobei $R^5$ einen Alkylrest mit 1 bis 4 Kohlenstoffatomen oder eine Phenylgruppe darstellt, die gegebenenfalls durch Fluor, Chlor, Trifluormethyl, Alkyl oder Alkoxy mit jeweils 1 bis 2 Kohlenstoffatomen substituiert ist,
zur Herstellung eines Arzneimittels zur Bekämpfung von Schädigungen des zentralen Nervensystems.

2. Verwendung von Verbindungen der allgemeinen Formel I gemäß Anspruch 1 zur Herstellung von Arzneimitteln zur Bekämpfung von Erkrankungen des zentralen Nervensystems, die auf einer Ischämie und/oder einer Hypoxie beruhen.

3. 2,6-Dimethyl-4-(3-nitrophenyl)-pyridin-3,5-dicarbon-säure-(2-hydroxyethyl)-isopropyl-ester.

4. Verfahren zur Herstellung von 2,6-Dimethyl-4-(3-nitrophenyl)-pyridin-3,5-dicarbonsäure-(2-hydroxyethyl)-isopropylester, dadurch gekennzeichnet, daß man 1,4-Dihydro-2,6-dimethyl-4-(3-nitrophenyl)-pyridin-3,5-dicarbon-säure-(2-hydroxyethyl)-isopropyl-ester mit oxidierenden (dehydrierenden) Agentien, gegebenenfalls in Gegenwart inerter Lösungsmittel, bei Temperaturen zwischen 0 und 200°C zur Reaktion bringt und das entstandene Oxidationsprodukt nach üblichen Methoden isoliert.

**Claims**

Use of compounds of the general formula I

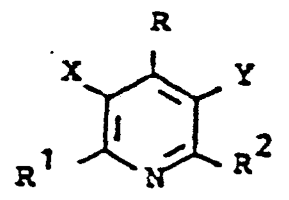

(I)

in which

R represents phenyl or pyridyl, the phenyl radical optionally being substituted once or twice by nitro, cyano, trifluoromethyl, fluorine, chlorine, bromine, iodine, alkyl, alkoxy, alkylmercapto having, in each case, 1 to 2 C atoms in the alkyl and alkoxy radicals,

$R^1$ and $R^2$ are identical or different and represent, in each case, hydrogen or an alkyl radical having 1 to 4 carbon atoms, which is optionally substituted by hydroxyl, or

$R^1$ together with X or $R^2$ together with Y together form a 5 to 7-membered ring, which is interrupted by carbonyl and optionally by oxygen and/or NH and

X and Y are identical or different and each represent a nitrile group or

X and Y represent the radical $-COR^3$, $R^3$ denoting an alkyl radical having 1 to 4 carbon atoms, phenyl or benzyl or X and Y represent the group $-COOR^4$, $R^4$ denoting hydrogen, a straight-chain, branched or cyclic saturated or unsaturated hydrocarbon radical having up to 10 carbon atoms, which is optionally interrupted by 1 oxygen atom in the chain and/or is optionally substituted by hydroxyl, fluorine, chlorine, bromine, cyano, acetoxy, pyridyl, phenyl or phenoxy, it being possible for the phenyl groups in turn to be optionally substituted by fluorine, chlorine, trifluoromethyl, nitro, alkyl having 1 to 4 C atoms or alkoxy having 1 to 4 C atoms, or

X and Y represent the group $-SO_2R^5$, $R^5$ representing an alkyl radical having 1 to 4 carbon atoms or a phenyl group, which is optionally substituted by fluorine, chlorine, trifluoromethyl, alkyl or alkoxy having, in each case, 1 to 2 carbon atoms,

for the preparation of a medicament for combating damage to the central nervous system.

2. Use of compounds of the general formula 1 according to Claim 1 for the preparation of medicaments for combating disorders of the central nervous system which are due to ischaemia and/or hypoxia.

3. 2,6-Dimethyl-4-(3-nitrophenyl)-pyridine-3,5-dicarboxylic acid (2-hydroxyethyl)-isopropyl ester.

4. Process for the preparation of 2,6-dimethyl-4-(3-nitrophenyl)-pyridine-3,5-dicarboxylic acid (2-hydroxyethyl)-isopropyl ester, characterised in that 1,4-dihydro-2,6-dimethyl-4-(3-nitrophenyl)-pyridine-3,5-dicarboxylic acid (2-hydroxyethyl)-isopropyl ester is reacted with oxidising (dehydrogenating agents), if appropriate in the presence of inert solvents, at temperatures between 0 and 200°C and the oxidation product formed is isolated by customary methods.

**Revendications**

1. Utilisation de composés de formule génerale

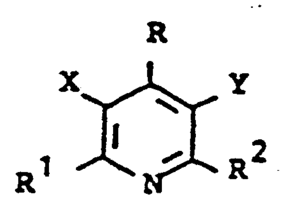

(I)

dans laquelle

R est un reste phényle ou pyridyle, le reste phényle portant éventuellement un ou deux substituants nitro, cyano, trifluorométhyle, fluoro, chloro, bromo, iodo, alkyle, alkoxy, alkylmercapto avec 1 ou 3 atomes de carbone dans chacun des restes alkyle et alkoxy,

$R^1$ et $R^2$ sont identiques ou différents et représentent chacun l'hydrogène ou un reste alkyle ayant 1 à 4 atomes de carbone, qui est éventuellement substitué par un radical hydroxy,

ou bien

$R^1$ conjointement avec X ou $R^2$ conjointement avec Y forment un noyau pentagonal à heptagonal qui est interrompu par un groupe carbonyle et le cas échéant par de l'oxygène et/ou un groupe NH et

X et Y sont identiques ou différents et représentent chacun un groupe nitrile

ou bien

représentent le reste $-COR^3$, dans lequel $R^3$ est un reste alkyle ayant 1 à 4 atomes de carbone, phényle ou benzyle,

ou bien

représentent le groupe $-COOR^4$ dans lequel $R^4$ est l'hydrogène, un reste d'hydrocarbure saturé ou insaturé, à chaîne droite, ramifié ou cyclique ayant jusqu'à 10 atomes de carbone, qui est interrompu le cas échéant par un atome d'oxygène dans la chaîne et/ou qui est substitué éventuellement par un radical hydroxy, fluoro, chloro, cyano, acétoxy, pyridyle, phényle ou phénoxy, les groupes phényle pouvant être substitués quant à eux le cas échéant par du fluor, du chlore, un reste trifluorométhyle, nitro, alkyle ayant 1 à 4 atomes de carbone ou alkoxy ayant 1 à 4 atomes de carbone

ou bien

représentent le groupe $-SO_2R^5$ dans lequel $R^5$ est un reste alkyle ayant 1 à 4 atomes de carbone ou un groupe phényle qui est éventuellement substitué par du fluor, du chlore, un substituant trifluorométhyle, alkyle ou alkoxy, avec dans chaque cas un ou deux atomes de carbone,

pour la préparation de médicaments destinés à combattre des troubles du système nerveux central.

2. Utilisation de composés de formule générale I suivant la revendication 1 pour la préparation de médicaments destinés à combattre des maladies du système nerveux central qui reposent sur une ischémie et/ou sur une hypoxie.

3. L'ester de (2-hydroxyéthyl)-isopropyle de l'acide 2,6-diméthyl-4-(3-nitrophényl)-pyridine-3,5-dicarboxylique.

4. Procédé de production de l'ester de 2-(hydroxyéthyl)-isopropyle de l'acide 2,6-diméthyl-4-(3-nitrophényl)-pyridine-3,5-dicarboxylique, caractérisé en ce qu'on fait réagir l'ester de (2-hydroxyéthyl)-isopropyle de l'acide 1,4-dihydro-2,6-diméthyl-4-(3-nitrophényl)-pyridine-3,5-dicarboxylique avec des agents oxydants (déshydrogénants), éventuellement en présence de solvants inertes, à des températures comprises entre 0 et 200° C et on isole par des opérations classiques le produit d'oxydation formé.